(19) Europäisches Patentamt
European Patent Office
Office européen des brevets

(11) Veröffentlichungsnummer: **0 206 298 B1**

# (12) EUROPÄISCHE PATENTSCHRIFT

(45) Veröffentlichungstag der Patentschrift: **20.04.88**

(51) Int. Cl.⁴: **C 07 C 121/34**, C 07 C 120/00

(21) Anmeldenummer: **86108490.3**

(22) Anmeldetag: **21.06.86**

## (54) Verfahren zur gemeinschaftlichen Herstellung von Ethylencyanhydrin und dessen Ethern.

(30) Priorität: **27.06.85 DE 3522906**

(43) Veröffentlichungstag der Anmeldung: **30.12.86 Patentblatt 86/52**

(45) Bekanntmachung des Hinweises auf die Patenterteilung: **20.04.88 Patentblatt 88/16**

(84) Benannte Vertragsstaaten: **BE DE FR GB IT NL**

(56) Entgegenhaltungen:
**DE - A - 2 121 325**
**DE - A - 2 655 794**
**DE - B - 1 189 975**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Hutmacher, Hans-Martin, Dr., Rüdigerstrasse 70, D-6700 Ludwigshafen (DE)**
Erfinder: **Hettinger, Peter, Dr., Schloss-Strasse 3, D-6803 Edingen-Neckarhausen (DE)**
Erfinder: **Voges, Dieter, Dr., Richard-Wagner-Strasse 28, D-6800 Mannheim 1 (DE)**
Erfinder: **Lengsfeld, Wolfgang, Dr., Woogstrasse 46, D-6703 Limburgerhof (DE)**

EP 0 206 298 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Ethylencyanhydrin (Ia) sowie gemeinschaftlich damit zur Herstellung von Ethern dieser Verbindung der allgemeinen Formel Ib

$$R{-}O{-}CH_2{-}CH_2{-}CN \qquad Ib$$

in welcher R für einen organischen Rest, vorzugsweise für einen Kohlenwasserstoffrest mit 1-6 C-Atomen steht.

Ethylencyanhydrin (Ia) sowie dessen Ether (Ib) sind bekanntermassen wichtige Zwischenprodukte für Synthesen.

Während die Ether durch Addition von Alkoholen R−OH (II) an Acrylnitril problemlos zugänglich sind (s. z.B. DE-OS 21 21 325), bereitet die analoge Umsetzung des Acrylnitrils mit Wasser zu Ia erhebliche technische Schwierigkeiten, da diese Reaktion bevorzugt zur Bildung des 2,2'-Dicyandiethylethers (III)

$$NC{-}CH_2{-}CH_2{-}O{-}CH_2{-}CH_2{-}CN \qquad III$$

führt (s. z.B. DE-AS 11 89 975), es sei denn, man verwendet einen unverhältnismässig grossen und damit unwirtschaftlichen Überschuss an Wasser (JP-OS 9196-850).

III lässt sich zwar in Gegenwart basischer Katalysatoren thermisch in Ia und Acrylnitril spalten (JP-OS 83/185 550), jedoch bereitet hierbei die verlustfreie Wiedergewinnung des zur Polymerisation neigenden Acrylnitrils erhebliche Mühe.

Nach einem weiteren Verfahren (s. z.B. DE-OS 26 55 794) wird die Direktsynthese von Ia aus Acrylnitril und Wasser unter Mitverwendung von Formaldehyd vorgenommen, jedoch gibt der Formaldehyd zu Nebenreaktionen Anlass und ausserdem gestaltet sich die Aufarbeitung des Reaktionsgemisches wegen des Formaldehydes und des nicht umgesetzten Acrylnitrils technisch schwierig.

Da bei all diesen Methoden die Gefahr erheblicher Verluste an Acrylnitril besteht, lag der Erfindung in erster Linie die Aufgabe zugrunde, das Ethylencyanhydrin auf wirtschaftlichere Weise herzustellen als bisher. Weiterhin beruht die Erfindung auf der Aufgabe, die Ether Ib verfahrenstechnisch einfach zu gewinnen.

Demgemäss wurde ein Verfahren zur gemeinschaftlichen Herstellung von Ethylencyanhydrin (Ia) und dessen Ethern der allgemeinen Formel Ib

$$R{-}O{-}CH_2{-}CH_2{-}CN \qquad Ib$$

in der R für einen organischen Rest steht, gefunden, welches dadurch gekennzeichnet ist, dass man einen Alkohol R−OH (II) in Gegenwart einer Base mit 2,2'-Dicyandiethylether (III) umsetzt.

Die Ausgangsverbindung III ist auf einfache Weise durch Umsetzung von Acrylnitril mit Wasser in Gegenwart einer Base zugänglich, beispielsweise nach dem Verfahren der DE-AS 11 89 975. Da es ein besonderer Vorteil des erfindungsgemässen Verfahrens ist, dass III nicht aus den Reaktionsgemischen seiner Herstellung, die neben III noch Wasser, die Base und Acrylnitril enthalten, isoliert zu werden braucht, geht man vorzugsweise von derartigen Reaktionsgemischen aus und versetzt diese mit dem Alkohol II, zumal in beiden Reaktionsschritten, der Herstellung von III und der Herstellung von Ia und Ib, die gleiche Base verwendet werden kann. Diese Verfahrensweise empfiehlt sich auch deswegen, weil die Umsetzung des Acrylnitrils nicht vollständig zu sein braucht, denn das überschüssige Acrylnitril wird beim erfindungsgemässen Reaktionsschritt glatt in Ib überführt.

Setzt man III in Abwesenheit von Acrylnitril um, erhält man Ia und Ib gemäss der Gleichung

$$NC{-}CH_2{-}CH_2{-}O{-}CH_2{-}CH_2{-}CN \xrightarrow{ROH} HO{-}CH_2{-}CH_2{-}CN + RO{-}CH_2{-}CH_2{-}CN$$

in etwa gleichen Ausbeuten, bezogen auf III, und ist Acrylnitril zugegeben, so erhöht sich bei genügendem Alkohol-Angebot die Ausbeute an Ib entsprechend.

Das gute Gelingen des erfindungsgemässen Verfahrens ist von der Art des Alkohols II prinzipiell nicht abhängig. Als Alkohole seien beispielsweise genannt:

- gesättigte und ungesättigte aliphatische Alkohole mit 1-20, vorzugsweise 1-6 C-Atomen, wie Methanol, Ethanol, Isopropanol und Allylalkohol;
- gesättigte und ungesättigte cycloaliphatische Alkohole, vorzugsweise solche mit 5 oder 6 Ringgliedern im cycloaliphatischen Rest, z.B. Cyclohexanol und Menthol;
- araliphatische Alkohole mit 7-20 C-Atomen, beispielsweise Benzylalkohol und Zimtalkohol;
- aromatische Alkohole, vorzugsweise ein- oder zweikernige Alkohole wie Phenol, die Kresole und die Naphthole.

Diese Alkohole können ferner Substituenten tragen, welche sich unter den Reaktionsbedingungen inert verhalten, also beispielsweise Halogen, $C_1-C_4$-Alkoxygruppen, $C_2-C_6$-Acylgruppen, tertiäre Aminogruppen, die Nitrogruppe und die Cyangruppe. Schliesslich kann man auch von mehrbasischen Alkoholen II ausgehen, beispielsweise von Ethylenglykol, wobei man je nach den Mengenverhältnissen die entsprechenden Hydroxyether oder Bis-ether erhält.

Für eine vollständige und hinreichend schnelle Spaltung des Ethers III, empfiehlt es sich, den Alkohol II in einer Menge von 0,5-5 mol/mol III einzusetzen.

Besondere Bedeutung hat das Verfahren für die Coproduktion von Ethylencyanhydrin und 3-Methoxypropionitril durch Umsetzung von III mit Methanol, da diese beiden Verbindungen besonders wichtige Zwischenprodukte für organische Synthesen sind.

Für die basenkatalysierte Etherspaltung von III kommen grundsätzlich beliebige Basen in Betracht, also z.B. die Hydroxide, Carbonate, Alkoholate und die Salze schwacher Säuren von Alkali- und Erdalkalimetallen, wobei NaOH und KOH bevorzugt werden. Daneben eignen sich tertiäre

Amine wie Triethylamin und Pyridin sowie basische Phasentransferkatalysatoren wie quartäre Ammonium- und Phosphoniumbasen wie Benzyl-trimethyl-ammoniumhydroxid und -phosphoniumhydroxid. Die basischen Phasentransferkatalysatoren verwendet man vor allem dann, wenn sie bereits im Umsetzungsgemisch des Acrylnitrils mit Wasser zu III vorliegen und man solche Gemische für die erfindungsgemässe Reaktion einsetzt.

Die Basen können prinzipiell in beliebiger Menge angewendet werden, denn diese hat lediglich einen Einfluss auf die Reaktionsgeschwindigkeit. Gute Ergebnisse erzielt man in der Regel mit 0,1-50, besonders 0,5-10 mval pro Mol III.

Die erfindungsgemässe Umsetzung verläuft bereits bei einer Temperatur von etwa 40° C mit hinreichender Geschwindigkeit und höhere Temperaturen als 140° C bringen in der Regel keinen Vorteil mehr. Vorzugsweise nimmt man die Reaktion bei etwa 50-120° C vor, wobei man u.U. unter erhöhtem Druck arbeiten muss.

Für die Herstellung von III aus Acrylnitril und Wasser empfehlen sich Temperaturen von 20-100, vorzugsweise 50-80° C.

Im übrigen bietet das erfindungsgemässe Verfahren keine verfahrenstechnischen Besonderheiten, so dass nähere Angaben hierzu entbehrlich sind; das gleiche gilt für die Aufarbeitung der erhaltenen Reaktionsgemische.

*Beispiel*

Herstellung von Ethylencyanhydrin und dessen Methylether

Eine Mischung aus

168 g (1,36 mol) 2,2'-Biscyanethylether (III)
46 g (0,9 mol) Acrylnitril
12 g (0,17 mol) Ethylencyanhydrin
27 g (0,008 mol) NaOH und
0,4 g (0,002 mol) Benzyl-trimethylammoniumhydroxid
16 g höhersiedende Substanzen

wie sie bei der Umsetzung von 212 g (4 mol) Acrylnitril mit 58 g (3,2 mol) Wasser und den genannten Mengen der Basen bei 65-70° C nach der Reaktionszeit von 1 h anfiel, wurde mit 640 g (20 mol) Methanol 3 h bei 70° C gerührt.

Aus der gaschromatographischen Analyse des Reaktionsgemisches ergibt sich, dass sich III nahezu vollständig zu gleichen molaren Teilen an Ethylencyanhydrin und 3-Methoxypropionitril umgesetzt hatte. Bezogen auf das ursprünglich eingesetzte Acrylnitril hatten sich somit rund 40% Ethylencyanhydrin und rund 48% von dessen Methylether gebildet.

## Patentansprüche

1. Verfahren zur gemeinschaftlichen Herstellung von Ethylencyanhydrin (Ia) und dessen Ethern der allgemeinen Formel (Ib)

$$R-O-CH_2-CH_2-CN \qquad Ib$$

in der R für einen organischen Rest steht, *dadurch gekennzeichnet*, dass man einen Alkohol R−OH (II) in Gegenwart einer Base mit 2,2'-Dicyandiethylether (III) umsetzt.

2. Verfahren nach Anspruch 1, *dadurch gekennzeichnet*, dass man III in Form von Reaktionsgemischen einsetzt, wie sie bei der Umsetzung von Acrylnitril mit Wasser in Gegenwart einer Base anfallen.

3. Verfahren nach den Ansprüchen 1 und 2, *dadurch gekennzeichnet*, dass man als Alkohol II einen aliphatischen Alkohol mit 1-6 C-Atomen verwendet.

## Claims

1. A process for the joint preparation of ethylene cyanohydrin (Ia) and its ethers of the general formula (Ib)

$$R-O-CH_2-CH_2-CN \qquad Ib$$

where R is an organic radical, wherein an alcohol R−OH (II) is reacted with 2,2'-dicyanodiethyl ether (III) in the presence of a base.

2. A process as claimed in claim 1, wherein III is used in the form of a reaction mixture obtained in the reaction of acrylonitrile with water in the presence of a base.

3. A process as claimed in claims 1 and 2, wherein an aliphatic alcohol of 1 to 6 carbon atoms is used as the alcohol II.

## Revendications

1. Procédé pour la préparation simultanée d'éthylènecyanhydrine (Ia) et de ses éthers de formule générale (Ib)

$$R-O-CH_2-CH_2-CN \qquad Ib$$

dans laquelle R est mis pour un radical organique, caractérisé en ce qu'on fait réagir un alcool R−OH (II) avec le 2,2'-dicyanodiéthyléther (III) en présence d'une base.

2. Procédé selon la revendication 1, caractérisé en ce qu'on utilise III sous forme de mélanges réactionnels tels qu'obtenus dans la réaction de l'acrylonitrile avec l'eau en présence d'une base.

3. Procédé selon la revendication 1 ou 2, caractérisé en ce qu'on utilise, en tant qu'alcool II, un alcool aliphatique à 1-6 atomes de C.